(19) Europäisches Patentamt
European Patent Office
Office européen des brevets

(11) **EP 3 023 093 B1**

(12) **EUROPEAN PATENT SPECIFICATION**

(45) Date of publication and mention
of the grant of the patent:
**26.07.2017 Bulletin 2017/30**

(51) Int Cl.:
*A61K 35/02* (2015.01)          *A61K 9/00* (2006.01)
*A61K 47/36* (2006.01)          *A61K 9/10* (2006.01)

(21) Application number: **15194697.7**

(22) Date of filing: **16.11.2015**

(54) **MEDICINAL PREPARATION BASED ON DIOSMECTITE**

MEDIZINISCHE ZUBEREITUNG AUF DER BASIS VON DIOSMECTIT

PRÉPARATION MÉDICINALE À BASE DE DIOSMECTITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.11.2014 IT MI20142008**

(43) Date of publication of application:
**25.05.2016 Bulletin 2016/21**

(73) Proprietor: **S.I.I.T. S.r.l.-Servizio Internazionale Imballaggi Termosaldanti**
**20090 Trezzano sul Naviglio MI (IT)**

(72) Inventors:
• **COSTA, ANDREA**
**20090 TREZZANO SUL NAVIGLIO (MI) (IT)**
• **MARCELLONI, LUCIANO**
**20090 TREZZANO SUL NAVIGLIO (MI) (IT)**
• **MADARO, ELENA**
**20090 TREZZANO SUL NAVIGLIO (MI) (IT)**
• **DOMINONI, MIRKO**
**20090 TREZZANO SUL NAVIGLIO (MI) (IT)**

(74) Representative: **Bianchetti Bracco Minoja S.r.l.**
**Via Plinio, 63**
**20129 Milano (IT)**

(56) References cited:
**EP-A1- 2 386 289      EP-B1- 2 405 890**
**US-A- 4 942 042**

**Description**

The present invention relates to a medicinal preparation containing diosmectite for the treatment of diarrhoea. More specifically, the invention relates to a ready-to-use oral composition taking the form of an aqueous suspension containing diosmectite and karaya gum, and a medicinal preparation whereby said aqueous suspension is packaged in sachets.

**Background to the invention**

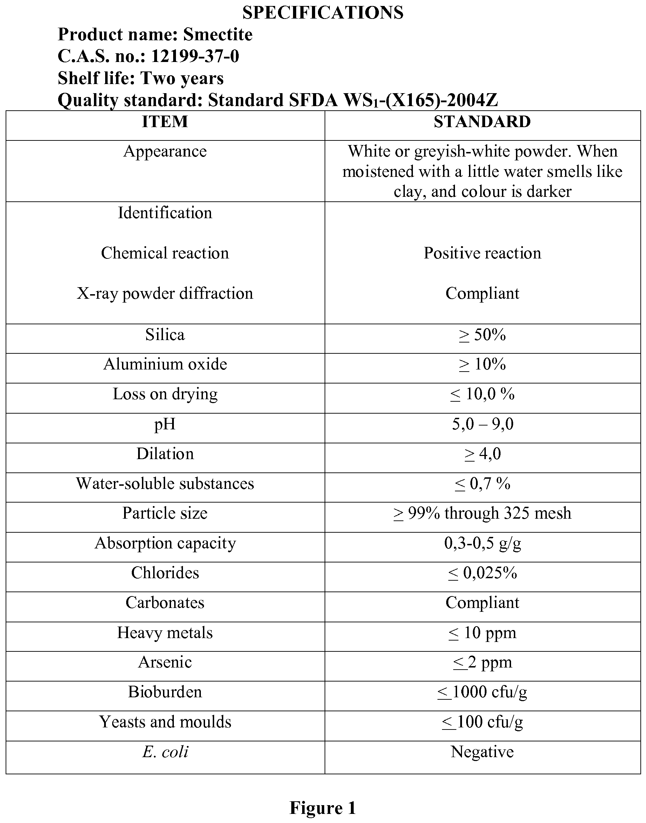

Diosmectite (or smectite) is a noble clay consisting of a natural silicate of magnesium and aluminium having a layered structure, wherein each layer consists of an octahedral sheet of aluminium sandwiched between two tetrahedral sheets of silicon. Fig. 1 shows the chemico-physical characteristics of diosmectite, in a form thereof available on the market under the name "Smectite".

This natural clay is widely used as an inert medical device to treat diarrhoea, due to its mechanical ability to drain and absorb many of the harmful substances present in large quantities in the altered intestinal environment, such as microbial toxins, excess water, electrolytes, bile acids, viruses and the like (Lin F. H. et al., Biomaterials, 2002, 23, 1981-1987; Carretero M. I. et al., Appl Clay Sci, 2002, 21, 155-163; Choy J.-H. et al., Appl Clay Science 2007, 36, 22-132; Cornejo J. et al., Clays Clay Miner, 1983, 31, 109-112; Ferrand T. et al., Appl Clay Sci, 1991, 6, 21-38; Poensin D. et al., J Bone Spine, 2003, 70, 367-370).

No other clay has demonstrated an absorbent capacity comparable to that of diosmectite; it indirectly protects the mucosal barrier against attack by toxic substances, aiding the restoration of the bowel function. The presence of clay also improves the rheological characteristics of the mucus, making it more difficult for toxic substances such as proteolytic enzymes and bile acids to pass through it.

Diosmectite absorbs large amounts of water, swelling up to twice its own volume, and binds cations from the surrounding soil due to the negative charges present on the surface of each crystalline cell. Numerous studies conducted *in vitro* and *in vivo* have demonstrated that diosmectite deactivates toxins of microbial origin, normalising intestinal transit times and preventing damage to the apical cells of the intestine. It has been demonstrated that the mineral neutralises the production of entero- and endotoxins by various pathogens such as *E. coli, Clostridium difficile*, *Clostridium perfringens*, *Bacteroides fragilis*, *Vibrio colerae* and mycobacteria in culture broth (Fioramonti J. et al., Toxicol Lett, 1987, 36, 227-232; Kheroua O. et al., Gastroenterol Clin Biol, 1987; 11, 240A; Navetat H. et al., Bull Acad Veterin Fr, 1988, 61, 365-75; Rateau J.G. et al., Curr Med Res Opin, 1982, 8, 224-241; Martirosian G. et al., Acta Microbiol Pol, 1998, 47(2), 177-83; Weese J.S. et al., Eq Veter J, 2010, 35(7), 638-641).

Due to the high surface area (SSA), amounting to 800 $m^2$ per gram, and the extremely small size of the crystals, diosmectite binds to the glycoproteins of the digestive mucus, causing changes in the chemical structure of the mucopolysaccharides and modifying their rheological properties. These changes greatly reduce penetration by bacterial toxins and bile acids through the layer of mucus, and the hydrolytic action of pepsin. As a result of these properties diosmectite can effectively absorb many other organic molecules, forming stable intercalation complexes.

Not only water but also many other molecules, especially large organic molecules, can be effectively absorbed in the interlayer region of the smectites, forming stable intercalation complexes.

After administration, diosmectite remains in the intestinal lumen, wherein it performs its effects without crossing the gastrointestinal barrier. The compound is then completely eliminated through the faeces. No side effects have been reported in clinical trials conducted with diosmectite; it has also been observed that diosmectite has very low toxicity (the TDL0 per os is 3500 mg/kg).

Various preparations in sachets for the treatment of diarrhoea are present on the market which contain, as main ingredient, about 3 g of powdered diosmectite for extempore reconstitution with water. Patent application EP 2386289 discloses an aqueous suspension of micronized diosmectite homogenized by agitation in the treatment of diarrhoea. In view of the sudden onset of attacks of diarrhoea it is desirable to have a ready-for-use suspension of diosmectite, with no need to add further water, in single-dose transportable containers. Said preparation should remain stable over time, guaranteeing the same pharmacological activity as the reference product. Moreover, when treated in bulk during the industrial manufacturing process, the clay component of the aqueous suspension must not sediment, which would hinder transfer of the mass being processed and filling of the single-dose containers, as well as giving rise to a non-homogenous product unsuitable for use.

**Description of figures**

Figure 1:    Specifications of smectite

Figure 2:     Schematic representation of high-pressure homogenisation

Figure 3:     Dimensional populations of a ready-for-use suspension of different samples of diosmectite according to the invention, on the basis of homogenisation pressure

Figure 4:     Comparative test of osmotic treatment of diosmectite water at different percentage concentrations, with and without the addition of 0.3% karaya gum.

Figure 5:     Desorptivity of different preparations of diosmectite

**Description of the invention**

The present invention achieves all the objectives listed above through the preparation of an aqueous suspension containing diosmectite and karaya gum, wherein the diosmectite particles are maintained within a given size range. It has been found that by adjusting the mean geometric diameter (MGD) of diosmectite to a value ranging between 2 $\mu$m and 20 $\mu$m, an aqueous suspension is obtained that remains stable to sedimentation over time and can easily be processed in bulk and packaged in suitable single-dose containers. It has also surprisingly been found that the addition of karaya gum significantly increases the ability of the aqueous suspension of diosmectite to absorb free water.

Quite unexpectedly, it was found that the product according to the invention is more effective than the reference product, consisting of diosmectite to be reconstituted at the time of use, in terms of ability to retain water and the substances present in it, such as electrolytes, thus providing a useful aid to the treatment of both acute and chronic diarrhoea.

The object of the present invention is therefore an oral composition containing diosmectite in aqueous suspension, karaya gum and optionally, pharmaceutically acceptable excipients, wherein the MGD of the diosmectite particles ranges between 2 $\mu$m and 20 $\mu$m, preferably between 2 $\mu$m and 7 $\mu$m.

Two techniques are available to homogenise a suspension of clay, both based on reducing the dimensions of the suspended phase. They stabilise the interactions between the continuous phase and particulate phase by means of a cutting action performed by turbines or blades rotating at high speed (turboemulsifiers or blenders), or by direct application of a pressure that forces the material to pass through narrow openings at high pressure (French presses, piston-driven homogenisers).

This second technology is preferred for the purposes of the present invention, as it has the advantage of not introducing air, which generates foam in the preparation.

During homogenisation it was observed that the reduction in size of the diosmectite particles generates a considerable increase in viscosity, which counteracts the tendency of the particles to sediment during the manufacturing process and in the packaged end product.

Said size reduction was monitored with the laser diffraction system using PIDS technology as described in example 1, whereby particle distribution can be determined on a large dimensional scale (from 0.04 to 2000 $\mu$m) by measuring the volume diameter (dV), defined as the diameter of a sphere having the same volume as the particle.

The MGD is defined as the mean geometric diameter of the particles present in the sample analysed, and is calculated with the following formula, starting with diameters dV of the particles present in the sample analysed:

$$DGM = \sqrt[n]{(dV_1 \, dV_2 \, dV_3 \ldots\ldots.dV_n)}$$

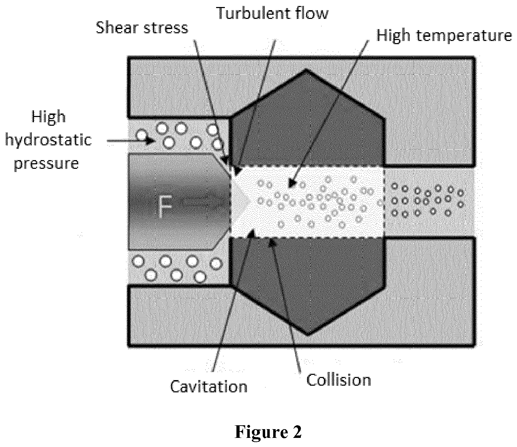

To obtain an MGD in the desired interval, the diosmectite can be advantageously subjected to the action of a high-pressure homogeniser according to the prior art, represented schematically in Figure 2. Using this technique the flow of material is forced through a narrow opening, operating at pressures ranging between 10 and 400 bars with one a or two complete treatment cycles having a duration that varies according to sample size and the pressure applied, and a flow rate which, depending on the size of the apparatus, ranges between 9 litres/hour and 9 litres/minute. In this case there is no need to thermostat at a particular temperature, because the size of diosmectite particles can be reduced very easily at low pressures.

Karaya gum is a dry exudate obtained from the trunk and branches of the tree *Sterculia urens,* native to India and Pakistan.

Karaya gum is normally used as a thickener, stabiliser and gelling agent, for pharmaceutical and food use.

In chemical terms, karaya gum is a rhamnogalacturonan (acid polysaccharide) of about 160 kDa containing about 40% D-galacturonic acid and 8-14% acetyl groups. Karaya gum has very low solubility in water (<0.02%). Higher concentrations of up to 5% produce highly viscous colloidal dispersions. Solutions of karaya gum are slightly acid (pH about 5), depending on the relative content of the acetyl groups.

Karaya gum possesses anti-inflammatory, soothing and antibacterial properties and is also resistant to bacterial and enzymatic degradation.

In a preferred embodiment of the present invention, karaya gum is present in concentrations ranging from 0.1% to 3%, preferably 0.3%, of the final weight of the composition.

The composition can also contain, as excipients, thickeners or viscosity-increasing agents, in particular gum tragacanth or gum arabic.

It has surprisingly been found that the addition of karaya gum at concentrations ranging from 0.1% to 3%, preferably 0.3%, of the final weight of the formulation, further increases the absorption efficacy of the aqueous suspension of diosmectite.

Other excipients which can be added to the aqueous suspension of diosmectite according to the invention include pH-adjusting agents (preferably citric acid), wetting agents (preferably sucrose esters), flavourings and preservatives.

In a preferred embodiment the composition according to the invention contains (% by weight) 19% diosmectite, 0.3% karaya gum, 0.25% citric acid, 0.15% sucrose esters, and water (q.s. for 100). 0.3% flavouring and 0.2% preservative can also be added.

The diosmectite composition according to the invention can be prepared as follows:

1) Diosmectite is added, under continuous mechanical stirring, to purified water placed in a suitable container.
2) Sucrose esters, acidifiers, preservatives and flavourings are added sequentially at 5-minute intervals.
3) The product is placed in the loading hopper of the homogeniser and subjected to size reduction.
4) The product is discharged and karaya gum is added; the resulting product is then mixed slowly for 30 minutes.

The suspension thus obtained is stable and homogenous and can be used to fill suitable single-dose containers.

Another aspect of the invention relates to a medicinal preparation containing a suspension of diosmectite as described herein, packaged in sachets, glass or plastic bottles, plastic vials and microenemas devices.

A further aspect of the invention relates to the use of the composition and medicinal preparation as described herein for the treatment of chronic and acute diarrhoea.

The following examples illustrate the invention in greater detail.

**EXAMPLES**

**Example 1 - Reduction in particle size of diosmectite**

A ready-for-use suspension of diosmectite was prepared (this Basic Formula contains about 19%), having the following general formula:

| Ingredients | mg / 1 sachet | % |
|---|---|---|
| Purified water | 12700.707 | 79.936 |
| Diosmectite | 3001.247 | 18.889 |
| Acid (citric) | 39.718 | 0.250 |
| Sucrose esters (wetting agent) | 23.831 | 0.150 |
| Flavouring | 45.000 | 0.283 |
| Preservative | 30.000 | 0.189 |
| Karaya gum | 48.000 | 0.302 |

The preparation was subjected to the action of a high-pressure piston-driven homogeniser for all ingredients except karaya gum, which was added at the end under slow stirring.

The suspension was subjected to treatment in said machine under different homogenisation pressure conditions, ranging from a minimum of 50 bars to a maximum of 200 bars' pressure, in various steps, and the size reduction obtained in each step was tested with a laser diffraction analyser.

The homogenisation conditions applied are shown in the table below.

| PRESSURE/RESISTANCE (bars) | NO. OF STEPS | Temperature (°C) |
|---|---|---|
| 50 | 1 | 24 |

(continued)

| PRESSURE/RESISTANCE (bars) | NO. OF STEPS | Temperature (°C) |
|---|---|---|
| 100/50 | 1 | 24 |
| 150/50 | 1 | 24 |
| 200/50 | 2 | 24 |

The absence of a temperature increase during the recirculations indicates complete transformation of the force applied in the particle reduction without dispersions of heat energy.

The effect of various different treatments on size reduction was measured with a Coulter model LS 13 320 laser diffraction system operating with PIDS technology, which rapidly determines the particle-size distribution on a large scale (from 0.04 $\mu$m to 2000 $\mu$m) with high resolution and reproducibility of the results, and calculates the "volume diameter". The volume diameter (dV) is defined as the diameter of a perfect sphere having the same volume as the particle under analysis.

The operational characteristics of the size measurements taken are as follows:

Optical path: 25.4 cm
Diffraction light source: solid state (780 nm)
Height: 45 cm
Humidity: 0% to 90%, non-condensing
Number of detectors: 132
Optical control: Auto-alignment
Particle-size analysis range: 0.4 $\mu$m to 2000 $\mu$m
Technology: light scattering, with complete implementation of both the Fraunhofer and Mei light-scattering theories
Temperature range: 10°-40°C
Typical analysis time: 15s-90 s

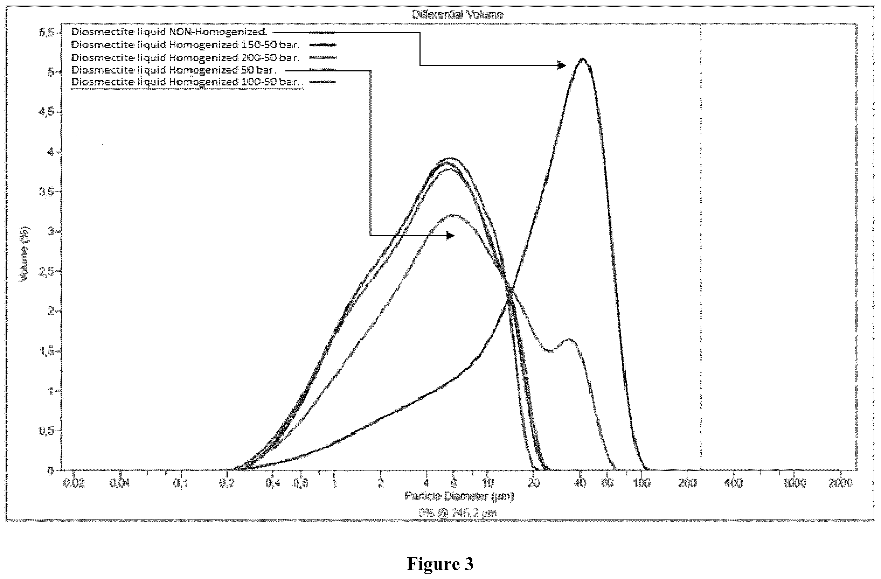

As will be seen in Figure 3, there are two particle-size populations: one further to the right (non-homogenised sample), with a median centred around 40 $\mu$m diameter, and one further to the left (homogenised samples), with a median around 5 $\mu$m, practically all with equivalent particle-size distributions for the samples subjected to 100/50, 150/50 and 200/50 bars.

The critical mean geometric diameter for obtaining a viscosity increase sufficient to prevent sedimentation of the preparation is 10.3 $\mu$m (at a pressure of 50 bars).

The suspension thus obtained remains stable to sedimentation for over 10 months from the test.

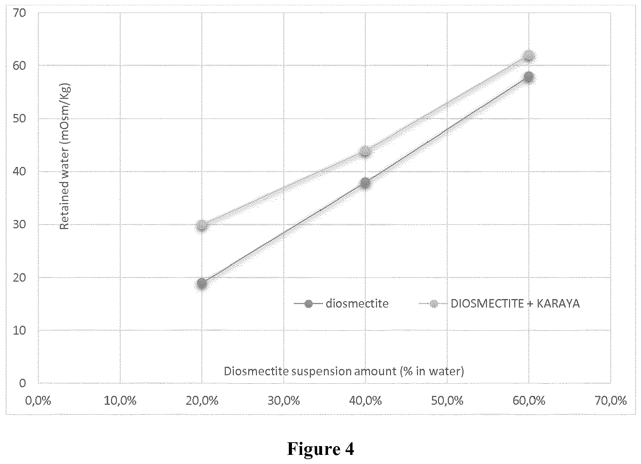

As shown in Fig. 4, the compositions containing karaya gum exhibited a better osmotic performance of the homogenised product with diosmectite at the usual use concentrations (15-40%) than the formulations not containing karaya gum, until the cohesion forces became predominant (80% in water).

Some composition examples are set out below.

**Example 2 - Compositions**

The following ready-for-use aqueous suspensions were prepared, in single-dose or multi-dose preparations suitable for oral administration, containing flavourings and sweeteners and having a pH ranging between 4 and 8. Preservatives and prebiotics can also be added to said compositions to support the restoration of the intestinal bacterial flora:

(1)

| Ingredients | mg/dose |
|---|---|
| Purified water | 11675 |
| Diosmectite | 3000 |
| Sucrose esters | 27 |
| Flavouring | 45 |
| Acesulfame K | 30 |

(continued)

| Ingredients | mg/dose |
|---|---|
| Potassium sorbate | 15 |

(2)

| Ingredients | mg/dose |
|---|---|
| Purified water | 10748 |
| Diosmectite | 3000 |
| Tartaric acid | 60 |
| Glycerin | 24 |
| Sodium saccharine | 6 |
| Chocolate flavouring | 45 |
| Methyl paraben | 30 |
| Karaya gum | 45 |

(3)

| Ingredients | mg/dose |
|---|---|
| Purified water | 9748 |
| Diosmectite | 3000 |
| Tartaric acid | 50 |
| Sucrose esters | 24 |
| Sucralose | 4 |
| Green apple flavouring | 47 |
| Fructooligosaccharides/Inulin | 2000 |
| Karaya gum | 35 |

**Example 3 - Bioassay**

The composition was evaluated in a system that simulates the conditions of gastrointestinal transit. A homogenate of diosmectite in aqueous suspension (homogenate), a homogenate in suspension with the addition of karaya gum (homogenate + karaya), and diosmectite powder for dissolving in a sachet (sachet currently on the market), were prepared for this purpose.

The reactor (human intestinal ecosystem simulator) was adapted from the SHIME System, which represents the adult human gastrointestinal tract (GIT), as described by Molly et al., Applied Microbiology and Biotechnology, 1993, 39, 254-258.

The SHIME consists of a succession of five reactors that simulate the various parts of the human gastrointestinal tract. The first two reactors simulate various stages, from food absorption to digestion, adding a given quantity of SHIME medium (140 mL 3 times/day), pancreatic and bile fluids (60 mL 3 times/day) to the stomach (V1) and duodenum (V2) respectively, with peristaltic pumps, on emptying of the respective reactors after specified intervals.

The absorption process that takes place in the small intestine is simulated by a dialysis tube situated between the small intestine and successive reactors that simulate the large intestine.

The last three compartments are continuously mixed at a constant volume with pH control.

The retention time and pH of the various sections are selected in such a way as to reproduce the *in vivo* conditions in the various parts of the gastrointestinal tract. The total residence time in the last three sectors, simulating the large intestine, is 72 hours.

After inoculation with faecal microbiota, said reactors simulate the ascending colon (V3), transverse colon (V4) and descending colon (V5). The preparation of the inoculum, retention time, pH, temperature and reactor feed composition have been described previously by Possemiers et al., FEMS Microbiology Ecology, 2004, 49, 495-507.

The samples collected during simulated transit through the gastrointestinal tract are used to analyse the water holding capacity (WHC) of the test product.

The method evaluates the bulking capacity of viscous and gelling products able to retain water in a simulated intestinal environment.

The method employed was based on suction pressure using dialysis tubes containing the test material in the simulated intestinal content (5 mL of fermenting content). To eliminate molecular weights over 2000 Da the dialysis tubes are immersed in a receptacle containing polyethylene glycol (PEG) to create osmotic suction that approximates water absorption in the colon. After a given time, the WHC is calculated as grams of water lost after drying divided by the residual dry weight.

The water holding capacity of different formulations was evaluated with a CST (capillary suction time) test. Capillary suction time studies of aqueous systems use the capillary suction pressure of porous paper. When a suspension is filtered under the influence of suction pressure, the rate at which the filtrate spreads from the suspension is controlled mainly by the filterability of the suspension.

The CST apparatus automatically measures the advance time between electrodes separated radially when a fixed area of special filter paper is exposed to the suspension.

The samples to be tested are placed in the sample cylinder, and the suction pressure of the filter paper under the sample extracts the filtrate.

The filtrate progresses radially in a substantially elliptical structure. The timer starts when the liquid reaches the first pair of electrodes, and the final time is recorded when the liquid reaches the third electrode.

The CST results measured were converted to desorptivity (S). Desorptivity characterises the water holding capacity of clay, and measures the physical efficacy of clay in treating attacks of diarrhoea by absorbing the water and positive ions in it and not releasing them.

Desorptivity (S) and water holding capacity are therefore inversely proportional, according to the following equation:

$$T_2 - T_1 = \left( \frac{\varepsilon h}{a^2 S} \right) (R_2^4 - R_1^4)$$

wherein T is the time at which the wetting front reaches radius $R$, "$\varepsilon$" is the porosity of the filter to water, "$h$" is the thickness of the filter, "$a$" is the inner radius of the filter and cell, and S is the desorptivity.

*Results of study*

The samples were collected from each incubation at the end of the stomach, halfway along the intestine and at the end of the small intestine (the cases respectively numbered I, II and III below). 3 preparations were examined:

(B) Ready-for-use suspension - A viscous homogenised product, without separation, containing 3000 mg of diosmectite and having a qualitative composition like formulation (3) in example 2.
(C) Extempore suspension - Reference product on the market, containing 3000 mg of diosmectite; solid in sachet, separates rapidly after reconstitution.
(D) Ready-for-use suspension + karaya gum - Homogenised product + suspending agent containing 3000 mg of diosmectite like preparation (B), plus 45 mg of karaya gum, and having a qualitative composition like formulation (4) in example 2.

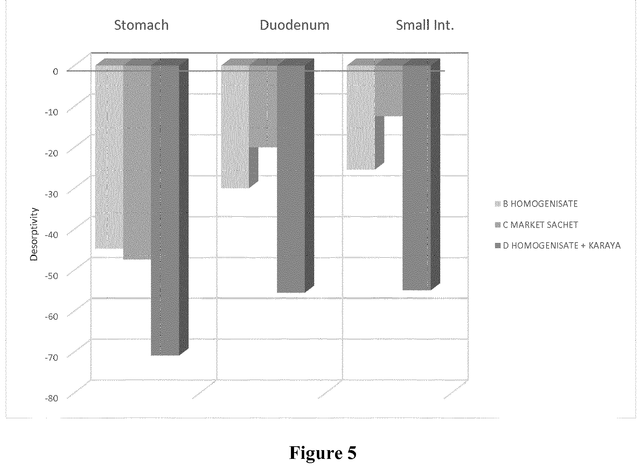

The results of the CST test were converted to desorptivity. Desorptivity indicates how easily the product tested releases water; consequently, the lower the desorptivity value the better the performance of the product. The data are presented as the % difference in desorptivity compared with the control (baseline without the addition of any product) (Figure 5). The results are shown in the Table below.

**Table: Water holding capacity of the various test products expressed as % difference in desorptivity compared with the control condition.**

| | B HOMOGENISATE | | C MARKET SACHET | | D HOMOGENISATE + KARAYA | |
|---|---|---|---|---|---|---|
| | MEAN | SD | MEAN | SD | MEAN | SD |
| STOMACH 1.5 h | -44.8 | 4.4 | -47.4 | 6.3 | -70.9 | 2.1 |
| DUODENUM 1.5 h | -30 | 5.0 | -20.0 | 4.8 | -55.6 | 4.3 |
| SMALL INTESTINE 3.0 h | -25.5 | 5.7 | -12.4 | 3.5 | -55.0 | 1.2 |

The differences in performance compared with the reference product in sachets were evaluated by ANOVA.

Product (D) gave significantly better results than reference product (C) in terms of water holding capacity during transit through the upper gastrointestinal tract.

It also performed significantly better than untreated ready-for-use product (B).

In terms of water holding capacity, the following order of water holding efficacy can be established:

(D) > (B) > (C) commercial reference product.

The results obtained in the small intestine were surprisingly always in favour of diosmectite in ready-for-use suspension with the addition of karaya gum.

**Claims**

1. Oral formulation in the form of an aqueous suspension, comprising:

   (a) diosmectite having a mean geometric particle diameter ranging from 2 $\mu$m to 20 $\mu$m;
   (b) karaya gum;
   and optionally,
   (c) physiologically compatible excipients.

2. Composition according to claim 1, wherein said mean geometric particle diameter ranges from 2 $\mu$m to 7 $\mu$m.

3. Composition according to claims 1 and 2, containing 0.1 to 3% by weight of karaya gum.

4. Composition according to claim 3, containing 0.3% by weight of karaya gum.

5. Composition according to claim 1, wherein said excipients are selected from viscosity-increasing agents, pH-adjusting agents, wetting agents, flavourings and preservatives.

6. Formulation according to claim 5, wherein said pH-adjusting agent is citric acid.

7. Formulation according to claim 5, wherein said wetting agent is a sucrose ester.

8. Medicinal preparation containing a composition according to claims 1-7 packaged in a sachet.

9. Composition according to claims 1-7, or medicinal preparation according to claim 8, for use in the treatment of acute or chronic diarrhoea.

**Patentansprüche**

1. Orale Formulierung in der Form einer wässrigen Suspension, umfassend:

   (a) Diosmectit mit einem mittleren geometrischen Teilchendurchmesser im Bereich von 2 $\mu$m bis 20 $\mu$m;

(b) Karaya-Gummi;
und optional,
(c) physiologisch kompatible Hilfsstoffe.

**2.** Zusammensetzung nach Anspruch 1, wobei der mittlere geometrische Teilchendurchmesser im Bereich von 2 $\mu$m bis 7 $\mu$m liegt.

**3.** Zusammensetzung nach den Ansprüchen 1 und 2, enthaltend 0,1 bis 3 Gew.-% Karaya-Gummi.

**4.** Zusammensetzung nach Anspruch 3, enthaltend 0,3 Gew.-% Karaya-Gummi.

**5.** Zusammensetzung nach Anspruch 1, wobei die Hilfsstoffe ausgewählt sind aus Viskositäts-erhöhenden Mitteln, pH-Wert-einstellenden Mitteln, Benetzungsmitteln, Geschmacksstoffen und Konservierungsmitteln.

**6.** Formulierung gemäß Anspruch 5, wobei das pH-Wert-einstellende Mittel Zitronensäure ist.

**7.** Formulierung gemäß Anspruch 5, wobei das Benetzungsmittel ein Sucrose-Ester ist.

**8.** Medizinisches Präparat, enthaltend eine Zusammensetzung nach den Ansprüchen 1 bis 7, verpackt in einen Portionsbeutel.

**9.** Zusammensetzung nach den Ansprüchen 1 bis 7 oder medizinisches Präparat nach Anspruch 8 zur Verwendung bei der Behandlung von akutem oder chronischem Durchfall.

**Revendications**

**1.** Formulation orale sous la forme d'une suspension aqueuse, comprenant :

(a) de la diosmectite ayant un diamètre de particule géométrique moyen compris entre 2 $\mu$m et 20 $\mu$m ;
(b) de la gomme de karaya ;
et éventuellement,
(c) des excipients physiologiquement compatibles.

**2.** Composition selon la revendication 1, dans laquelle ledit diamètre de particule géométrique moyen est compris entre 2 $\mu$m et 7 $\mu$m.

**3.** Composition selon les revendications 1 et 2, contenant 0,1 à 3 % en poids de gomme de karaya.

**4.** Composition selon la revendication 3, contenant 0,3 % en poids de gomme de karaya.

**5.** Composition selon la revendication 1, dans laquelle lesdits excipients sont choisis parmi les agents augmentant la viscosité, les agents d'ajustement de pH, les agents mouillants, les agents aromatisants et les conservateurs.

**6.** Formulation selon la revendication 5, dans laquelle ledit agent d'ajustement de pH est l'acide citrique.

**7.** Formulation selon la revendication 5, dans laquelle ledit agent mouillant est un ester de saccharose.

**8.** Préparation médicinale contenant une composition selon les revendications 1 à 7, emballée dans un sachet.

**9.** Composition selon les revendications 1 à 7, ou préparation médicinale selon la revendication 8, pour une utilisation dans le traitement de la diarrhée aiguë ou chronique.

**SPECIFICATIONS**

**Product name: Smectite**
**C.A.S. no.: 12199-37-0**
**Shelf life: Two years**
**Quality standard: Standard SFDA WS$_1$-(X165)-2004Z**

| ITEM | STANDARD |
|---|---|
| Appearance | White or greyish-white powder. When moistened with a little water smells like clay, and colour is darker |
| Identification<br><br>Chemical reaction<br><br>X-ray powder diffraction | <br><br>Positive reaction<br><br>Compliant |
| Silica | $\geq 50\%$ |
| Aluminium oxide | $\geq 10\%$ |
| Loss on drying | $\leq 10,0\,\%$ |
| pH | $5,0 - 9,0$ |
| Dilation | $\geq 4,0$ |
| Water-soluble substances | $\leq 0,7\,\%$ |
| Particle size | $\geq 99\%$ through 325 mesh |
| Absorption capacity | 0,3-0,5 g/g |
| Chlorides | $\leq 0,025\%$ |
| Carbonates | Compliant |
| Heavy metals | $\leq 10$ ppm |
| Arsenic | $\leq 2$ ppm |
| Bioburden | $\leq 1000$ cfu/g |
| Yeasts and moulds | $\leq 100$ cfu/g |
| *E. coli* | Negative |

**Figure 1**

**Figure 2**

**Figure 3**

**Figure 4**

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2386289 A

**Non-patent literature cited in the description**

- **LIN F. H. et al.** *Biomaterials,* 2002, vol. 23, 1981-1987
- **CARRETERO M. I. et al.** *Appl Clay Sci,* 2002, vol. 21, 155-163
- **CHOY J.-H. et al.** *Appl Clay Science,* 2007, vol. 36, 22-132
- **CORNEJO J. et al.** *Clays Clay Miner,* 1983, vol. 31, 109-112
- **FERRAND T. et al.** *Appl Clay Sci,* 1991, vol. 6, 21-38
- **POENSIN D. et al.** *J Bone Spine,* 2003, vol. 70, 367-370
- **FIORAMONTI J. et al.** *Toxicol Lett,* 1987, vol. 36, 227-232
- **KHEROUA O. et al.** *Gastroenterol Clin Biol,* 1987, vol. 11, 240A
- **NAVETAT H. et al.** *Bull Acad Veterin Fr,* 1988, vol. 61, 365-75
- **RATEAU J.G. et al.** *Curr Med Res Opin,* 1982, vol. 8, 224-241
- **MARTIROSIAN G. et al.** *Acta Microbiol Pol,* 1998, vol. 47 ((2)), 177-83
- **WEESE J.S. et al.** *Eq Veter J,* 2010, vol. 35 ((7)), 638-641
- **MOLLY et al.** *Applied Microbiology and Biotechnology,* 1993, vol. 39, 254-258
- **POSSEMIERS et al.** *FEMS Microbiology Ecology,* 2004, vol. 49, 495-507